# EUROPEAN PATENT SPECIFICATION

(11) **EP 3 355 900 B1**
(45) Date of publication and mention of the grant of the patent: **26.05.2021**
(21) Application number: 15890159.5
(22) Date of filing: 09.07.2015
(51) Int. Cl.: A61K 36/28, A61P 17/00, A61P 31/10, A61P 37/00

(54) **A GYNURA PROCUMBENS OIL EXTRACT FOR THE TREATMENT OF SEVERAL DERMATOLOGICAL DISEASES AND ITS PROCESS THEREOF**
GYNURA-PROUMBENS-ÖLEXTRAKT ZUR BEHANDLUNG MEHRERER DERMATOLOGISCHER ERKRANKUNGEN UND VERFAHREN DAFÜR
EXTRAIT HUILEUX DE GYNURA PROCUMBENS POUR LE TRAITEMENT DE PLUSIEURS MALADIES DERMATOLOGIQUES ET PROCÉDÉ CORRESPONDANT

(43) Date of publication of application: 08.08.2018
(73) Proprietor: Kostas, Grigoriou, M., D., 3027 Limassol (CY)
(72) Inventor: Kostas, Grigoriou, M., D., 3027 Limassol (CY)
(74) Representative: Costa, Persa
(86) International application number: PCT/CY2015/000001
(87) International publication number: WO 2017/005226

(56) References cited:
- CN-A- 103 961 301
- CN-C- 100 490 780
- ISKANDER M N ET AL: "Antiinflammatory screening of the medicinal plant Gynura procumbens", PLANTS FOODS FOR HUMAN NUTRITION, KLUWER ACADEMIC PUBLISHERS, NL, vol. 57, no. 3-4, 1 October 2002 (2002-10-01), pages 233-244, XP009192469, ISSN: 0921-9668
- KAEWSEEJAN N ET AL: "Evaluation of Phytochemical Composition and Antibacterial Property of Gynura procumbens Extract", ASIAN JOURNAL OF PLANT SCIENCES, ASIAN NETWORK FOR SCIENTIFIC INFORMATION, JP, vol. 11, no. 2, 1 February 2012 (2012-02-01), pages 77-82, XP009192471, ISSN: 1682-3974

## Description

### INTRODUCTION

Plant products today symbolize safety in contrast to synthetic drugs.'

Gynura procumbens is an annual evergreen shrub from the family of asteroecae with a fleshy stemand purple tint. Gynuraprocumbensisfound in various parts inAsia and is widely used in this countries as a traditional medicine for hundreds of years .This plant is used in the traditional treatment of various health aliments such eruptive fevers, rash, kidney disease, migraines, constipation, hypertension, diabetes mellitus, and cancer.² Gynura oil extract is also content in Chinese cosmetic creams (patent No CN 100490780 C). The literature search suggests that leaves or leave extracts has anti herpes simplex virus* , antihyperclygaemic⁴, antihyperglygaemic and anthyperlipidaemic⁵ anti-inflammatory⁶, anticancerogennic⁷, blood hypertension reduction capabilities , antiproliferative on human mesangial cell, antioxidative¹⁰ and anticancerogenic" properties.
1 GII, N.S., PSharma, J., Bahwa, K. Dhlman;., S.Sood, P.D.Sharma and M. Bail, 2010. Study of cucumls melo var.utilissimus seeds of the therapeutic potential. J. Plant SC., S; 248-255.
2 Perry, L.M., 1980. Medicinal plants of east and south east Asia: Attributed properties and uses. 1st Edn., The MIT press, Cambridge, MassachusetU and London, ISBN 0-262-16076-5, pp334-360.
3 Nawawl, A., N.Nakamura, M.Hattory,M.Kurokawa and K.Shiraki, 1999. Inhibitory effects of Indonesian medicinal plantsonthe Infection of herpes simplex virus type 1.Phytoth. Res, 13:37-44.
4 Ll et al., 2009, Akowuah et al., 2009
S Zhang and Tan, 2000
6 Islander M.N., Y. Song, I.M. Coopar and W.J Iratcharlyakul, 2002. Anti-inflammatory screening of the medicinal plant Gynura procumbens. Plant Foods Hum. Nut. 57:233-244.
7 Agustina, D., SM Haryana and A.Supartinah, 2006. Anticarcinogenesis ePect of gynura procumbens (Lour)merron tongue carcinogenesis in 4NQO-induced rat Dental J., 39:126-132.
8 Klm, M Lee, J, S. Wiryowldagdo and H.K. Kim, 2006.Antihypenensive effects of gynura procumbens extracton spontaneously hypertensive rats.1.Med. Food, 9:587-590.
9 Lee, Hu., Chung, S. Wlryowldagdo, W. Chun, S Kim and M. Choe, 2007. Inhibitory effects of an aqueus extract extract of gynura procumbens on human mesangial cell proliferation .Korean J. Physiol.Pharmacol., 11:145-148.
10 Puangpronpltag et al., 2010; Rosldah et at.,2008,2009
11 Mahmood. A.A,. A.A Marlod, F. Al Bayaty and S.A Abdel-Wahab, 2010. Anti-ulcerogenic activity of gynura procumbens leaf extract against experimentally Induced gastric lesions In rats. J. Med. plants Res,. 4:685-691.

Due to the regenerative properties of gynura procumbens, it was decided to create an oil extract from the gynura procumbens leaves and the test of it in vivo forvariousskin diseases.

### DETAILED DESCRIPTION OF THE INVENTION

The present invention provides an alternative and innovative method for treating Onychom-icosis and Vitiligo using the oil extract from Gynura procumbens as topical administration. The said composition is extracted from semi dried leaves from a plantation in Limassol, created for this purpose.

The following embodiments and descriptions are to be construed as merely illustrative,

The term "generalized vitiligo" as used herein, refers to the condition in which the pigment is lost from at least 40% of the skin surface.

The term "Newly formed vitiligo" refers to the condition in which the pigment is lost from some areas of the skin in less than 2 years.

Theterm "oldformed vitiligo" as used herein, refers to the condition in which the pigment is lost from some areas of the skin for over 2years.

Polyphenols contained in gynura procumbens leaves are bioactive constituents very important in the control and prevention of tissue damage by activating oxygen species due to their antioxidant effect. Phenolcompounds scavenge the catastrophic for the cellsfree radicals.
12 Lanauri et at.

Reactive free radicals including superoxide, hydroxyl radical and peroxyl radical generally result indegradation of protein, lipid peroxidation and oxidation of DNA.'³
13 Oanls, T,G., Maderla, V.M., and Almalda L.M., (1994)Action of phanolic derivativas as inhibitors of membrane lipid peroxidaton and as paroxyl radical scavengers.

Skin consists of three main structural parts, the epidermis, the dermis and the hypodermis. The main cells of theepidermis layer are: keratinocytes, Iangerhans cells and melanocytes. Main cells ofthe dermis are fibroblasts producing collagen, elastin and structural proteoglycans, mast cells and macrophages. The skin is protected by the acid mantle, a slightly acid film on the surfaceoftheskinwhich acts as a barrierto bacteria viruses and otherpotential contaminants that might penetrate the skin. The ph ofthe skin is between 4, 5 and 6, 2 and this acidity helps to neutralize the primarily alkaline nature of contaminants. Sebaceusglands lipids makean important contribution in maintaining the integrity ofthe skin barrier and express pro inflammatory and anti-inflammatory properties.

Regulation of the function of keratinocytes due to the free radicals scavenging activity of the gynura extract regulates and activates the primary function of them which is the formation of the barrier against the environmental damage of pathogenic bacteria, viruses, fungi, parasites, heat, UV radiation and water loss.

Neutralizing the effect of the free radicals by the gynura oil extract in the hypodermis langerhans cells-their function is the production of microbial antigens - activates and causes them to become fully functional antigen presenting cells.

Melanocytes function is to insert granules of melanin into specialized cellular vesicles called melanosomes, and by them melanin is transferred into the other skin cells of the human epidermis. The melanosomes in each recipient accumulate atop the cells nucleus protecting the nuclear DNA from mutations caused by ionizing radiation ofthe suns ultraviolet rays. Activation ofthe affected melanocytes due to polyphenols free radical scavenging activity of the gynura extract gives more protection to the cells against UV radiation.'⁴
14 Brenner M, Hearing U.J (2008) Tha protective role of melanin against UV damage in human skin.

Mast cells (mastocytes) are a part of the immune system. They are known for their role in allergy and anaphylaxis and also play an important and protective role involved in wood healing, including allergy and angiogenesis.¹⁵ By neutralizing the effects of the free radicals on the affected mast cells, the said cells become fully functioned cells.
IS Jung M et al., (1 February 2013) <BiologicalChemistry 288(5):3289-3304

By regulating the function of fibroblasts, the affected cells are stimulated, thus producing more collagen, increasing the natural protection of the skin.

Neutralizing the destructive action of the free radicals by using the gynura extract, the cells are released, their functions are regulated, and all protective mechanisms are activated and thus become able to neutralize pathogenic factors.

### PREPARATION OF GYNURA PROCUMBENS OIL EXTRACT

The method introduced herein for the preparation of the gynura procumbens oil extract comprises of drying the leaves in a certain amount of time, heating the leaves in ester for a certain amount of time followed by filtration.

In order to maximize the potency of the said extract 4 variables have been tested.
1) The time in which the extract is heated
2) The temperature in which the extract is heated
3) The time in which the leaves are dried
4) The ester/leaves ratio

The temperatures selected for the for the 1" variable were 35°C, 50°C, 65°C, 80°C and 100°C. The time selected for the 2'^{d} variable was 2,4,6,8, 12 hours. Each of these was tested separately..

The sample with the greatest potency and an efficient oil infusion was observed when the sample was heated for 8 hours at 65°C.

The biggest difference in the potency of the gynura procumbens oil extract was the amountof time the leaves were dried. Two samples were tested in 2 groups for the treatment of onychomycosis comprising of 15 volunteers where in sample A the leaves were completely dried and in sample B the leaves were semi dried. A and B samples were heated for 8 hours at 65°C.

Sample A had very little medicinal properties when applied topically where sample B had close to 90% cure rate.

Based on the above information and results, the final method for creating a gynura procumbens oil extract was formulated along with a clinical study to screen its efficiency.

Due to the low molecular weight of polyphenols contained in the leaves of gynura procumbens, an ester with a low molecular weight is used to increase the efficiency of the free radical scavenging activity. Isopropyl myristate is a sufficient ester for this purpose.

### Process

1. Washing one kg of fresh leaves with running water, and then spreading it out indoors on a buzzard fabric which is changed daily to absorb moisture at a room temperature for seven days.
2. Soaking the semi dried leaves in 70% ethanol for sterilization, for 30 minutes.
3. Removing the leaves from ethanol and putting them in a sterile stainless steel container for 30 minutes.
4. Adding one kg of isopropyl myristate and then sealing it with a sterile cap.
5. Incubating in a laboratory thermostat for 8 hours at 65°C.
6. Filtering the extract

### New approach for the treatment of onychomycosis by gynura oil extract

The nail consists of the nail plate, the nail matrix, and the nail bed below it. The nail bed is the skin below the nail plate. The nail matrixis responsible for producingcells becoming the nail plate. The matrixwill continue to grow as long as it receives nutrition and remains ina healthy condition. The bed below the matrix contains all the cells of the epidermis and the dermis which are keratinocytes, langerhans cells, melanocytes, mast cells, and fibroblasts. Neutralizing the function of the free radicals by the gynura oil extract, the function of the affected cells in the bed below the matrix of the nail, recovers and all protective mechanisms activate and neutralize the pathogenic factors.

A clinical study was conducted in order to screen the efficiency of the gynura procumbens oil extract. The primary efficacy endpoint was the proportion of patients achieving "Responder rate" at the end of the treatment. The secondary efficiacy endpoint was the proportion of patients achieving "complete cure".

### SCORING CLINICAL INDEX FOR ONYCHOMYCOSIS IN ONYCHOMYCOSIS TREATING WITH GYNURA PROCUMBENS OIL EXTRACT

Overall, 132 patients were included in the efficacy analysis. At baseline, the percentage of the affected target nail area was in average 75%.
Study type: Local Condition: Onychomycosis
Intervention: gynura procumbens oil extract

### Description

Gynura procumbens oil extract (as produced) was topically administered on the nail plate and around the plate area. The said oil extract wastopically administered twice per day. Total duration of treatment was 4 to 9 months.

### Participant flow: overall study

| | Gynura procumbens oil extract |
|---|---|
| Started | 132 |
| Treated | 128 |
| Completed | 114 |
| Not completed | 18 |
| Lost to follow up | 4 |
| Failed to Visit Doctor | 5 |
| Participant NON-compliance | 9 |

### Baseline measures

| | Gynura procumbens oil extract |
|---|---|
| Number of Participants [units:participants] | 132 |
| Female | 61 |
| Male | 71 |

### Outcome measures

Number of participants who showed no improvement During the clinical study, 3 participants out of 114 showed no improvement.

Number of participants whoshowed up to 35% improvement 4 participants out of 114 showed improvement in the following stages.
From 0-2 months
   0% of the infected nail plate is cured
From 2-4 months
   1S% of the infected nail plate is cured
From 4-6 months
   1S-2S% of the infected nail plate is cured
From 6-8 months
   30% of the infected nail plate is cured
From 8-10 months
   35% of the infected nail plate is cured

Number of participants showing 35-60% improvement 4 participants out of 114showed improvement in the following stages.
From 0-2 months
   10% of the infected nail plate is cured
From 2-4 months
   20% of the infected nail plate is cured
From 4-6 months
   40% of the infected nail plate is cured
From 6-8 months
   50% of the infected nail plate is cured
From 8-10 months
   60% of the infected nail plate is cured

Number of participants who showed 60-85% improvement 12 participants out of 114 showed improvement in the following stages
From 0-2 months
   20% of the infected nail plate is cured
From 2-4 months
   50% of the infected nail plate is cured
From 4-6 months
   55% of the infected nail plate is cured
From 6-8 months
   60% of the infected nail plate is cured
From 8-10 months
   65% of the infected nail plate is cured

Number of participants showing 85-100% improvement 91 participants out of 114 showed improvement in the following stages
From.0-2 months
   50% of the infected nail plate is cured
From 2-4 months
   70% of the infected nail plate is cured
From 4-6 months
   75% of the infected nail plate is cured
From 6-8 months
   80% of the infected nail plate is cured
From 8-10 months
   85%+ of the infected nail plate is cured
From 114 patients from who received treatment, 80 patients were completely cured. The time necessary to achieve complete treatment ranged from 4-10months.

The results indicate that it generally takes 3-8 months to reach significant improvement or complete cure. After 1-2 months the nail root is able to produce healthy nail cells whilst the fungus is eliminated.

It is worth mentioning that Onychomycosis is extremely difficult to treat because the causative fungal infection resides in the nail bed, underneath the tough, keratinous nail plate. Current topical medications lack potency and the abilityto penetrate the nail plate, there by greatly limiting efficacy. Current oral drugs carry the risk of serious side effects.

The only FDA-approved topical treatment yields complete cure rates of 5.5% to 8.5% after one year of treatment (Penlac package insert). Systemic oral medications for onychomycosis yield cure rates ranging from 14% to38% (Lamisil and Sporonox package inserts) but may require hepatic assessment and monitoring and may cause various adverse reactions, drug-drug interactions, and toxicities due to the doses required to concentrate therapy at the actual site of infection.

### ACTIVATING AFFECTED MELANOCYTES. NEW APPROACH FOR THE TREATMENT OF VITILIGO

Vitiligo is a chronic disease characterized by portions of the skin losing their pigment. It occurs when skin cells die or are unable to function. Aside from cases contact with chemicals, the cause ofvitiligo is unknown. Researchers suggest that vitiligo may arise from autoimmune, genetic, oxidative stress, neural or viral causes.'⁶ Until now, an effective treatment for vitiligo has not been reported.
Z6 Charter, R M; Chappal, JL (2009) Vitiligo update.

Vitiligo is typically classified into two main categories: segmental and non-segmental vitiligo. The global incidence of vitiligo is less than 1% with some populations averaging between 2-3% and as high as 16%. Autoimmune diseases such as Addison disease, Hashimotos thyroiditis, and type 1 diabetes mellitus tend to occur more often in people who have vitiligo.* Treatment: There is no cure of vitiligo but many treatment options are available. The best evidence is for applied steroids and the combination of ultraviolet light in combination with creams."

Topical preparations of immune suppressing medications including glucocorticoids and calcineurin inhibitors (such as tacrolimus and pimecrolimus) are considered to be first line vitiligo treatments.
17 Kruger C Schallreuter KU (October 2012)A review of worldwide prevalence of vitiligo In children adolascan U and adults.
18 Whitton, ME;Ashkroft, DM;Gonzales, U (2008) Therapeutic Interventions

Psoralen and ultraviolet A (PUVA) treatment involves taking a drug increasing the skin sensitivity to ultraviolet light, then exposing the skin to high doses of UVA light.

Psoralen and ultraviolet- A light (PUVA) treatment has serious risks such as
1. Skin cancer and cancer. Exposure to PUVA treatment may result in skin cancer
2. Skin damage. Exposure to UV light may lead to sunburn and skin damage
3. Risk of cataracts
4. Other skin diseases getting worse

Due to the regenerative action of the polyphenols contained in the gynura procumbens oil extract, the melanocytes are released from the catastrophic free radical activity, increasing the melanin production. 8 volunteers received treatment for vitiligo, wherein one was a generalized vitiligo.

The method of treatment for Vitiligo comprised the topical administration of the gynura procumbens oil extract 2 times daily. Sunlight exposure for 30 minutes daily was advised.
8 patients received treatment for vitiligo where-
in 1 case was generalized vitiligo
   Over 80% of the affected areas had disappeared in 3 months
3 cases were newly formed vitiligo
   Over 60% of the affected areas had disappeared in 3 months
4 cases were old formed vitiligo
   Less than 10% of the affected areas had disappeared in 3 months

It is believed that by combining phototherapy with the topical administration ofthe gynura oil extract, the results will greatly improve.

### Conclusions

This invention proposes an alternative way for the treatment of onychomycosis and vitiligo. The said treatment comprising of topically applying the gynura procumbens oil extract, improves the ability of the human skin cells through activating their protective mechanisms by neutralizing the free radical scavenging activity, to fight effectively against pathogenic factors causing onycomycosis and vitiligo.

A safe, topical treatment yet more effective than oral therapy for onychomycosis is presented. The popular treatments for onychomycosis comprises of topical treatments that yield complete cure rates of 5.5% to 8.5% and systematic oral medications that yield cure rates ranging from 14% to 38%. It is assumed that the gynura procumbens oil extract is by far a superior treatment that yields an average success rate of 83.8% without any side effects.

In addition, an efficient and safe treatment for vitiligo is introduced without any adverse effects. It is believed that by combining light phototherapy with topical administration of gynura oil extract, a treatment that will excel most of not all currently available treatments will be introduced.

Moreover, considering the emergency of multiple drug resistance strains of microorganisms due to indiscriminate use of antibiotics and the severe side effects of antibiotics, antifungal and other preparations used in the classic medicine for the treatment of skin infections, the significance of the approach becomes more important.

## Claims

1. A method of achieving a gynura procumbens extract via the heating of semi dried leaves of gynura that have been dried in room temperature for seven days mixed with isopropyl myristate oil, after they have been washed with running water, left to dry then soaked in ethanol for half an hour for sterilization. The said extract is then filtered.

2. The method of claim 1 can be carried out by heating the semi dried leaves at a temperature between 40°C -100°C

3. The method of claim 1 includes the drying of the leaves in room temperature for seven days and can be carried out by using a thermostat or Calcium Chloride

4. The method of claim 1 includes the sterilization of the semi dried leaves and can be carried out by soaking the said leaves in ethanol for 1 to 20 minutes

5. The method of claim 1 includes infusing the semi dried leaves, wherein the said leaves have moisture content greater than 10% and less than 70%

6. The extract resulting from the method in claim 1 for use in the treatment of Onychomycosis

## Patentansprüche

1. Eine Methode einen Extrakt von Gynura Procumbens zu beschaffen, durch die Erwaermung von halbgetrockneten Gynurablaettern, die in Zimmertemperatur fuer sieben Tagen getrocknet wurden, gemischt mit Isopropyl Myristatoel, nachdem sie mit fliessendem Wasser gewascht worden waren, trocken lassen und dann in Ethanol fuer eine halbe Stunde zur Sterilisation einweichen.

2. Die Methode von Anspruch 1, kann durch Erwaermung der halbgetrockneten Blaetter, bei eine Temperatur zwischen 40° C - 100° C, erfuehlt werden.°

3. Die Methode von Anspruch 1, beinhaltet das Trocken von den Blaettern in Zimmertemperatur fuer sieben Tagen und kann, durch Benutzung eines Thermostates oder Calcium Chlorides, erreicht werden.

4. Die Methode von Anspruch 1, beinhaltet die Sterilisation von den halbgetrockneten Blaettern und kann durch erweichen der obengenannten Blaetter in Ethanol fuer 1 bis 20 Minuten erfolgt werden.

5. Die Methode von Anspruch 1, beinhaltet das Aufgiessen der halbgetrockneten Blaetter, wobei die obengenannten Blaetter einen Feuchtigkeitsgehalt von mehr als 10% und weniger als 70% haben.

6. Der Extract, der aus der Methode 1 entstehet zur Verwendung bei der Behandlung der Onychomykose.

## Revendications

1. Une méthode d'obtention d'un extrait de gynura procumbens via le chauffage de feuilles semi-séchées de gynura qui ont été séchées à température ambiante pendant sept jours mélangées avec de l'huile de myristate d'isopropyle, après avoir été lavées à l'eau courante, laissées à sécher puis trempées dans de l'éthanol pendant une demi-heure pour la stérilisation. Ledit extrait est ensuite filtré.

2. La méthode de la procédure 1 peut être effectuée en chauffant les feuilles semi-séchées à une température comprise entre 40°C -100°C.

3. La méthode de la procédure 1 comprend le séchage des feuilles à température ambiante pendant sept jours et peut être effectuée en utilisant un thermostat ou du chlorure de calcium.

4. La méthode de la procédure 1 comprend la stérilisation des feuilles semi-séchées et peut être réalisée en trempant lesdites feuilles dans de l'éthanol pendant 1 à 20 minutes.

5. La méthode de la procédure 1, comprend l'infusion des feuilles semi-séchées, dans lequel lesdites feuilles ont une teneur en humidité supérieure à 10% et inférieure à 70%.

6. L'extrait résultant de la méthode selon la revendication 1, destiné à être utilisé dans le traitement de l'onychomycose
